# EUROPEAN PATENT SPECIFICATION

(11) **EP 1 343 414 B1**
(45) Date of publication and mention of the grant of the patent: **16.02.2005**
(21) Application number: 01271175.0
(22) Date of filing: 19.12.2001
(51) Int. Cl.: A61B 5/00

(54) **ACTIVITY AID APPARATUS**
VORRICHTUNG ZUR UNTERSTÜTZUNG VON AKTIVITÄTEN
APPAREIL D'ASSISTANCE d'activité

(30) Priority: 19.12.2000 SE 0004710
(43) Date of publication of application: 17.09.2003
(73) Proprietor: Mandometer AB, 141 04 Huddinge (SE)
(72) Inventor: ZANDIAN, Michel, S-115 43 Stockholm (SE); BERGH, Cecilia, S-118 20 Stockholm (SE); SÖDERSTEN, Per, S-118 20 Stockholm (SE)
(74) Representative: Onn, Thorsten
(86) International application number: PCT/SE2001/002849
(87) International publication number: WO 2002/049508

(56) References cited:
- WO-A1-00/27274
- US-A- 4 101 071
- US-A- 5 263 491
- US-A- 5 704 350
- US-A- 6 135 950
- US-A1- 2001 049 470

## Description

### Technical field

The present invention pertains to a portable arrangement for correcting the amount of physical activity to a preferred level of dieting, and a method therefore.

### Background art

Many persons in our current society are in need of an aid for dieting in a controlled manner. These are, for example, athletics, overweighed, obese and others who have to care about a controlled way of dieting. A specific problem while dieting relates to a correct or suitable physical activity in combination with the dieting. People tend to overdo with more physical activity than required for a specific level of dieting.

A problem is that there are plenty of methods and devices on the market to keep a diet, such as athletic training equipment, drugs, designed food for dieting, associations for dieting etc. If a person dieting should be able to succeed in doing so and achieve an intended goal it should be supervised by people experienced in this art, for example, experts on nutrition, doctors and the like. Also, persons under diet should have an aid that they can rely on when they are without supervision from specific experts in the field.

It is known that a controlled diet gives an as good result as the combination of physical activity and dieting, at least for people with ordinary needs of physical activity, i.e., non athletics, who should combine dieting with ordinary daily activities such as a daily walk. Athletics on the other hand do have to take the same care of their body when preparing for major sporting events, whereby they have to be careful with their food intake in correlation with their training effort.

There are unfortunately also lethal diseases related to a wrong dieting and such related to unhealthy dieting, such as anorexia, bulimia, and disorders related to digestion or gastrointestinal discomfort. Therefore, people at risk and/or their doctors should appreciate an aid for a good and healthy dieting.

The US patent 5,817,006 by Bergh and Södersten provides a basis for the teaching of dieting in a controlled maner. It provides a system used to measure eating rate, whereby different rates of ingestion correspond to a biologically determined degree of satiety. Eating rate is measured utilizing the variables (weight of food, time). For the quantification of satiety the interval scale of Borg is used. The invention makes use of reference standards (standard curves or curves of normality), obtained from research on a population of individuals. These curves reflect the average rate of ingestion that has been found statistically significant through the investigation of groups, differing in, for example, age and weight, within the population of men and women. The interval scale of Borg is used to record satiety.

One aim of the invention according to the US patent 5,817,006 by Bergh and Södersten, is to develop a measuring device that allows, among others, obese people to gain access to a method for weight control. Overweight and obesity is a major health problem, and the overweight, therefore, have an obvious interest in such a device. Furthermore, the control of body weight and ingestive behaviour is of considerable importance to athletes and those engaged in sports. A method for the control of body weight and eating behaviour is also needed within clinical medicine to care for patients suffering from anorexia, bulimia and gastrointestinal discomfort, that is to say disorders related to the intake of food. Use of the present method and device is likely to be of importance to those within the general public who are at risk to develop disorders of body weight control.

A device, according to the US patent 5,817,006 by Bergh and Södersten, mentioned provides to assist in the control of body weight for the individual with a possibility to control eating behaviour and the perception of satiety and, therefore, a method to control body weight. This is accomplished by the display of the reference standards on a monitor/screen. The reference standards for eating behaviour and satiety as a function of eating rate are derived from biologically based mean values obtained through research on samples of individuals from the general population. Using this device, the individual is in a position to adapt its eating behaviour and perception of satiety to what, by definition, is normal through the selection of the proper reference values and by adapting its rate of ingestion to the reference value displayed on a medium, for example a monitor, in real time. The rate of ingestion of the individual is displayed simultaneously with the reference standard and the two are to overlap.

Persons participating in the development of the invention and displaying deviations from the reference standards with respect to eating behaviour and perception of satiety have considered themselves unable or ignorant as to how to eat and how to feel satiated.

However the Us patent 5,817,006 by Bergh and Södersten, does not teach how to correlate an efficient amount of physical activity to a certain level of dieting. Especially there is no teaching of how to avoid unnecessary physical activity in combination with the eating training.

### Summary of the disclosed invention

It is an aim of the present invention to provide an arrangement/apparatus and a method to be used for controlling the amount of physical activity when dieting. Hereby, it provides a lightweight body portable arrangement for directing the amount of physical activity to a corresponding preferred level of dieting. There is a close relation between dieting and a corresponding level of physical activity.

In general the following rule prevails throughout the present invention. For an obese person the body temperature should be lowered and the physical activity should be raised in order to lose weight, and for a person with low weight, the body temperature should be raised and physical activity should be reduced in order to gain or keep weight. Of course there are weights between obese and low weight where a fairly low weight person should lose weight.

The present invention thus proposes a lightweight body portable arrangement for correcting the amount of physical activity to a preferred level of dieting. It comprises:
at least one sensor attached to a body part of a human user, registering movements with a predetermined resolution of the movement of the body part;
a processor, having a memory connected, controlling and recording input signals from the sensor;
a comparator means, comparing the input signals with predetermined stored movements within a provided resolution for the preferred level of dieting in the memory; and
a feedback means providing an output signal to the user, whereby the output signal indicates how to adapt the movements to the stored movements, thus adapting physical body activity to a level corresponding to the dieting level, whereby physical activity is being correlated to the level of dieting.

In one embodiment of the invention it comprises that movements stored for the preferred level of dieting are correlated to at least one of the parameters weight and height of a human being.

Another embodiment comprises that the preferred stored level of movements for dieting are correlated to a human beings Body Mass Index.

A further embodiment comprises feedback for physical activity, through at least two signals, demanding to increase or decrease activity or movements, respectively.

A still further embodiment comprises that the signals are sound, visual display or tactile feedback signals.

Yet another embodiment comprises that a processor and the means mentioned are comprised in a portable housing with a display.

A yet further embodiment sets forth that the housing comprises at least one sensor.

A yet still further embodiment comprises that the predetermined stored movements differ between different activities.

Furthermore, the present invention sets forth a method using a body portable arrangement for correcting the amount of physical activity to a preferred level of dieting. Hereby, comprising the steps of:
attaching at least one sensor to a body part of a human user, registering movements with a predetermined resolution of the movement of the body part;
controlling and recording input signals from the sensor through a processor, having a memory connected;
comparing the input signals with predetermined stored movements within a provided resolution for the preferred level of dieting in the memory; and
providing a feedback through an output signal to the user, whereby the output signal indicates how to adapt the movements to the stored movements, thus adapting physical body activity to a level corresponding to the dieting level, whereby physical activity is being correlated to the level of dieting.

It is appreciated that the embodiments of the arrangement are provided in the method of the present invention in accordance with the attached set of method sub-claims.

### Brief description of the drawings

Henceforth reference is had to the attached drawings in context of the accompanying description for a better understanding of the present invention with its embodiments and given examples, wherein:
**Fig. 1** illustrates an arrangement for controlling physical activity during dieting in accordance with one embodiment of the present invention;
**Fig. 2** illustrates six diagrams of measured physical activity with a sensor in accordance with the present invention; and
**Fig. 3** illustrates a diagram of activity for a person with a low Body Mass Index.

### Table

A table is provided at the end of the present description illustrating counts made with a sensor for registering physical activity in accordance with the present invention.

### Detailed description of preferred embodiments

The present invention provides a new and inventive arrangement and a method for controlling physical activity during dieting in order to achieve a good mode of dieting.

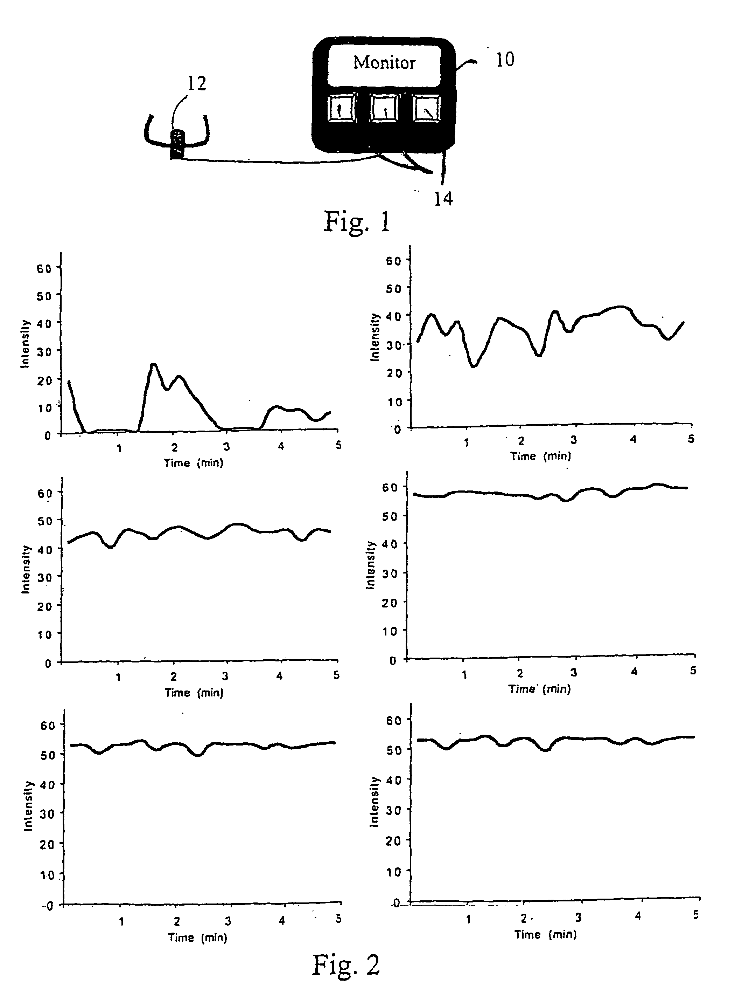

Fig. 1 schematically depicts a housing 10 comprising the arrangement provided by the present invention with a monitor or screen for the display of a current level of physical activity, for example, in measurements of movements made by a limb of a human being, having the housing with activity sensor 12 attached to the limb. The body part could, e.g. be an ankle, a wrist, whereby the sensor 12 is attached to a bracelet or the like in a known manner for a person skilled in the art, or to other body parts, for example, with adhesives. It is known in the art of signal transmission to transmit those signals wireless or through a wire connection between a sensor and a receiver to be computed by a processor which uses the obtained sensor signals for control purposes. The present invention is not restricted to use only one of those transmission methods in accordance with prior art.

The modus operandus of the present invention is not to measure an absolute amount of work done or counting calories based on activity, but more to revise activity levels based on monitoring human beings physical activity and simultaneously give them a feedback of the intensity of the level of physical activity using a learning paradigm.

The present invention thus proposes a lightweight body portable arrangement or apparatus 10, 12 for correcting the amount of physical activity to a preferred level of dieting. It comprises:
at least one sensor 12 attached to a body part of a human user, registering movements with a predetermined resolution, for example a predetermined time period, of the movement of the body part;
a processor, having a memory connected, controlling and recording input signals from the sensor;
a comparator means, comparing the input signals with predetermined stored movements within a provided resolution for the preferred level of dieting in the memory; and
a feedback means providing an output signal to the user. The output signal indicates how to adapt the movements to the stored movements, thus adapting physical body activity to a level corresponding to the dieting level. It is accomplished through the arrangement that physical activity is correlated to the level of a preferred dieting.

In one embodiment of the invention movements stored for a preferred level of dieting are correlated to at least one of the parameters weight and height of a human being. A preferred correlation is thus a human beings Body Mass Index (BMI).

Feedback for physical activity is provided through at least two signals in one embodiment, thus demanding through the signals to increase or decrease activity or movements, respectively. Providing two signals could prove to be essential due to the fact that it is not sufficient just to provide a visual display of the movements of a body part during a measured time period. A feedback signal to alert a person during an activity should be provided, because a simple visual signal is easily disregarded. Hence, at least two signals are provided, such as sound and visual display, or tactile feedback signals through vibrations and a visual display, or even al three mentioned. A sound or tactile feedback could be produced with different frequencies regarding lowering or increasing activity. Tactile feedback through vibrations and similar methods are well known in the art for the same, and not further described.

It is also appreciated that the predetermined stored movements of a body part differ between different activities.

The arrangement 10, 12 provided by the present invention is designed so that it provides simultaneous feedback on a current activity level. This is used in a learning paradigm (negative feedback method), and useful for those who are not aware of their activity levels in order to keep a corresponding diet. Hence, it promotes to revise activity-based behaviour. Learning through monitoring actual activity with feedback has been proved by studies of activity in accordance with the present invention to be more sufficient than just monitoring per se as a method to change and/or maintain a specific behaviour during activity. Thus, the arrangement and the method used in accordance with the present invention monitors current activity and provides feedback to help a subject to activity to distinguish between different kinds of such activities.

Preferably, the arrangement is designed to make a person using it aware of exceeding or falling below a determined activity level for its level of dieting, thus adjusting itself to a more accurate level of gaining or loosing weight. It is designed to be comfortable to bear and battery operated digitising, calculating and storing obtained sensor input signals with, for example, an LCD display and operation buttons 14. A visual display of motion samplings is provided by the LCD display or the like.

Furthermore, the present invention sets forth a method using a body portable arrangement for correcting the amount of physical activity to a preferred level of dieting. Hereby, comprising the steps of:
attaching at least one sensor to a body part of a human user, registering movements with a predetermined resolution of the movement of the body part;
controlling and recording input signals from the sensor through a processor, having a memory connected;
comparing the input signals with predetermined stored movements within a provided resolution for the preferred level of dieting in the memory. Hence, providing a feedback through an output signal to the user. The output signal indicates how to adapt the movements to the stored movements, thus adapting physical body activity to a level corresponding to the dieting level, whereby physical activity is being correlated to the level of dieting.

During a pre-study of the present invention different activity levels where monitored by the use of an activity sensor 12. The activity sensor did catch the intensity of several different physical activities, which average, low and high level values are depicted in the attached Table at the end of the present description. Six different activities where monitored for a human being with a specific determined diet level, namely, resting, housekeeping, walking, running, ascending stairs and descending stairs. Average, low and high values are as mentioned classified in the attached Table.

Fig. 2 illustrates the result of the sensor input in six diagrams each representing obtained motions for the specific type of activities mentioned in the table. The diagrams present a time scale, 0-5 minutes, on their x-axis and intensity, 0-60 motions, on their y-axis. Measurements registered with the sensor 12 make up the sum of counted motions (horizontal or vertical shaking) per 16 sec (one possible resolution among others) during 5 min in each diagram. Wen reading the diagrams the upper row of diagrams in Fig. 2 depicts the intensity versus time for resting in the diagram to the left and housekeeping to the right, the intermediate row depicts walking to the left and running to the right, and the lower row depicts ascending stairs to the left and descending stairs to the right.

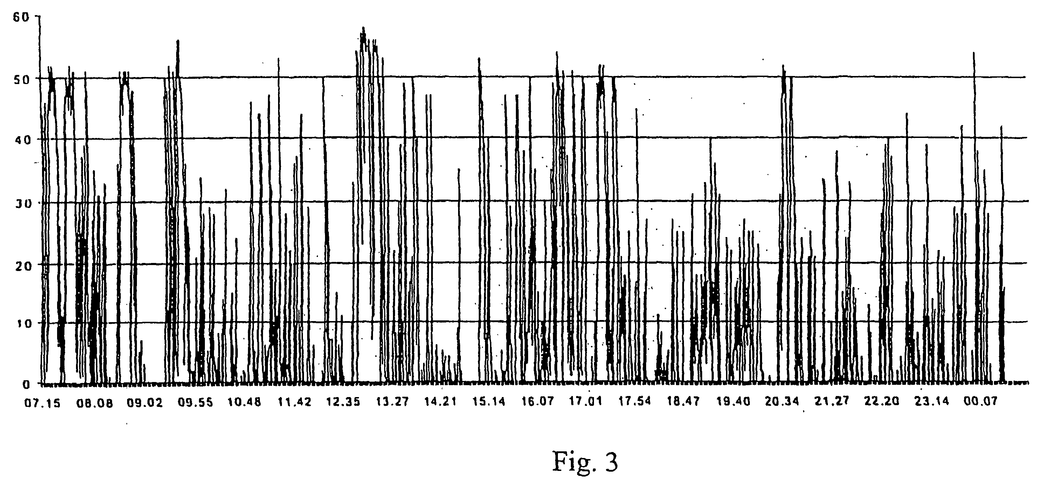

Fig. 3 illustrates a diagram with a graph showing the amount of physical body activity for a person with a low BMI of 14. Intensity of body motion, 0-60, is depicted on the y-axis and time, 07.15-00.07, on the x-axis. For this specific activity, the motion corresponding to the BMI should not be higher then 20 in intensity in order for the person to gain weight. Moreover every time period with motions exceeding 20 should be followed by a feedback in accordance with the present invention to alert the person of excessive motions.

It is appreciated that means not specifically named throughout present description are depicted from known software or hardware means or a combination of both known to a person skilled in the art.

While the arrangement and method shown or described has been characterized as being preferred it will be obvious that various changes and modifications may be made therein without departing from the scope of the invention as defined in the attached set of claims.

**Table**

| **Activity** | **Average** | **Low/High value** |
|---|---|---|
| Resting | 06,0 | 0-15 |
| Housekeeping | 34,00 | 30-40 |
| Walking | 44,00 | 42-48 |
| Running | 57,00 | 54-60 |
| Ascending stairs | 53,00 | 50-56 |
| Descending | 54,00 | 50-56 |

## Claims

1. A portable arrangement (10, 12) for correcting the amount of physical activity to a preferred level of dieting, comprising:
at least one sensor (12) attached to a body part of a human user, registering movements with a predetermined resolution of the movement of said body part;
a processor, having a memory connected, controlling and recording input signals from said sensor (12);
a comparator means, comparing said input signals with predetermined stored movements within a provided resolution for said preferred level of dieting in said memory; and
a feedback means providing an output signal to said user, whereby said output signal indicates how to adapt said movements to said stored movements, thus adapting physical body activity to a level corresponding to said dieting level, whereby physical activity is being correlated to said level of dieting.

2. An arrangement according to claim 1, wherein said movements stored for the preferred level of dieting is correlated to at least one of the parameters weight and height of said human being.

3. An arrangement according to claim 1, wherein said preferred stored level of movements for dieting is correlated to said human beings Body Mass Index.

4. An arrangement according to claims 1-3, wherein said feedback through at least two signals demands to increase or decrease movements, respectively.

5. An arrangement according to claim 4, wherein said signals are sound, visual display or tactile feedback signals.

6. An arrangement according to claims 1-5, wherein said processor and said means are comprised in a portable housing with a display.

7. An arrangement according to claim 6, wherein said housing comprises said at least one sensor.

8. An arrangement according to claims 1-7, wherein said predetermined stored movements differ between different activities.

9. A method using a body portable arrangement (10, 12) for correcting the amount of physical activity to a preferred level of dieting, comprising:
attaching at least one sensor (12) to a body part of a human user, registering movements with a predetermined resolution of the movement of said body part;
controlling and recording input signals from said sensor (12) through a processor, having a memory connected;
comparing said input signals with predetermined stored movements within a provided resolution for said preferred level of dieting in said memory; and
providing a feedback through an output signal to said user, whereby said output signal indicates how to adapt said movements to said stored movements, thus adapting physical body activity to a level corresponding to said dieting level, whereby physical activity is being correlated to said level of dieting.

10. A method according to claim 9, wherein said movements stored for the preferred level of dieting are correlated to at least one of the parameters weight and height of said human being.

11. A method according to claim 9, wherein said preferred stored level of movements for dieting is correlated to said human beings Body Mass Index.

12. A method according to claims 9-11, wherein said feedback through at least two signals demands to increase or decrease movements, respectively.

13. A method according to claim 12, wherein said signals are sound, visual display or tactile feedback signals.

14. A method according to claims 9-13, wherein said processor and said means are comprised in a portable housing with a display.

15. A method according to claim 14, wherein said housing comprises said at least one sensor.

16. A method according to claims 9-15, wherein said predetermined stored movements differ between different activities.

## Patentansprüche

1. Tragbare Anordnung (10, 12) zum Korrigieren des Ausmasses körperlicher Aktivität auf ein bevorzugtes Diät-Ausmass, enthaltend:
mindestens einen an einem Körperteil eines menschlichen Benutzers angebrachten Sensor (12), der Bewegungen mit einer vorgegebenen Auflösung der Bewegung des genannten Körperteils registriert;
einen Prozessor mit einem daran angeschlossenen Speicher, der Eingangssignale vom genannten Sensor (12) kontrolliert und aufzeichnet;
einen Komparator, der die genannten Eingangssignale mit vorgegebenen, in dem genannten Speicher gespeicherten Bewegungen innerhalb einer vorgesehenen Auflösung für das genannte, bevorzugte Diät-Ausmass vergleicht; und
ein Rückmeldungsmittel, das dem genannten Benutzer ein Ausgangssignal liefert, wodurch das genannte Ausgangssignal angibt, wie die genannten Bewegungen an die genannten gespeicherten Bewegungen anzupassen sind, und so die physische Körperaktivität auf ein dem genannten Diät-Ausmass entsprechendes Ausmass anpasst, wodurch die körperliche Aktivität mit dem genannten Diät-Ausmass korreliert wird.

2. Vorrichtung nach Anspruch 1, worin die für das bevorzugte Diät-Ausmass gespeicherten Bewegungen mit mindestens einem der Parameter Körpergewicht und Körpergrösse des genannten Menschen korreliert sind.

3. Vorrichtung nach Anspruch 1, worin das genannte bevorzugte gespeicherte Bewegungsausmass für die Diät mit dem Body Mass Index des genannten Menschen korreliert ist.

4. Vorrichtung nach den Ansprüchen 1-3, worin die genannte Rückmeldung über mindestens zwei Signale anzeigt, dass die Bewegungen zu verstärken bzw. zu verringern sind.

5. Vorrichtung nach Anspruch 4, worin die genannten Signale Töne, visuelle Anzeigen oder taktile Rückmeldungssignale sind.

6. Vorrichtung nach den Ansprüchen 1-5, worin der Prozessor und die genannten Mittel in einem tragbaren Gehäuse mit einer Anzeige enthalten sind.

7. Vorrichtung nach Anspruch 6, worin das Gehäuse mindestens einen Sensor aufweist.

8. Vorrichtung nach den Ansprüchen 1-7, worin sich die gespeicherten vorgegebenen Bewegungen je nach Aktivität unterscheiden.

9. Verfahren, unter Benutzung einer auf dem Körper tragbaren Vorrichtung (10, 12), zum Korrigieren des Ausmasses körperlicher Aktivität auf ein bevorzugtes Diät-Ausmass, beinhaltend:
das Anbringen mindestens eines Sensors (12) an einem Körperteil eines menschlichen Benutzers, wobei der Sensor Bewegungen mit einer vorgegebenen Auflösung der Bewegung des genannten Körperteils registriert;
das Kontrollieren und Aufzeichnen von Eingangssignalen von dem genannten Sensor (12) durch einen Prozessor mit einem daran angeschlossenen Speicher;
das Vergleichen der genannten Eingangssignale mit vorgegebenen, im genannten Speicher gespeicherten Bewegungen innerhalb einer vorgesehenen Auflösung für das bevorzugte Diät-Ausmass; und
die Bereitstellung einer Rückmeldung durch ein Ausgangssignal für den Benutzer, durch die das genannte Ausgangssignal anzeigt wie die genannten Bewegungen an die genannten gespeicherten Bewegungen anzupassen sind, und so die physische Körperaktivität auf ein dem genannten Diät-Ausmass entsprechendes Ausmass anpasst, wodurch die körperliche Aktivität mit dem genannten Diät-Ausmass korreliert wird.

10. Verfahren nach Anspruch 9, worin die für das bevorzugte Diät-Ausmass gespeicherten Bewegungen mit mindestens einem der Parameter Körpergewicht und Körpergrösse des Menschen korreliert sind.

11. Verfahren nach Anspruch 9, worin das genannte bevorzugte gespeicherte Bewegungsausmass für die Diät mit dem Body Mass Index des Menschen korreliert ist.

12. Verfahren nach den Ansprüchen 9-11, worin die genannte Rückmeldung über mindestens zwei Signale anzeigt, dass die Bewegungen zu verstärken bzw. zu verringern sind.

13. Verfahren nach Anspruch 12, worin die genannten Signale Töne, visuelle Anzeigen oder taktile Rückmeldungssignale sind.

14. Verfahren nach den Ansprüchen 9-13, worin der Prozessor und die genannten Mittel in einem tragbaren Gehäuse mit einer Anzeige enthalten sind.

15. Verfahren nach Anspruch 14, worin das Gehäuse mindestens einen Sensor aufweist.

16. Verfahren nach den Ansprüchen 9-15, worin sich die gespeicherten vorgegebenen Bewegungen je nach Aktivität unterscheiden.

## Revendications

1. Agencement portable (10, 12) pour corriger le montant d'activité physique à un niveau de régime alimentaire préféré, comprenant:
au moins un capteur (12) attaché à une partie du corps d'un utilisateur humain, qui enregistre des mouvements avec une résolution prédéterminée du mouvement de ladite partie du corps;
un processeur auquel est reliée une mémoire et qui contrôle et enregistre des signaux d'entrée fournis par ledit capteur (12);
un moyen comparateur qui compare lesdits signaux d'entrée à des mouvements prédéterminés à l'intérieur d'une résolution donnée enregistrés dans ladite mémoire pour ledit niveau de régime préféré; et
un moyen de rétroaction fournissant un signal de sortie audit utilisateur, ledit signal de sortie indiquant comment lesdits mouvements sont à adapter auxdits mouvements enregistrés, adaptant ainsi l'activité physique du corps à un niveau qui correspond audit niveau de régime, de sorte que l'activité physique est corrélée audit niveau de régime.

2. Agencement selon la revendication 1, où lesdits mouvements enregistrés pour le niveau de régime préféré sont corrélés à l'un au moins parmi les paramètres poids et taille dudit être humain.

3. Agencement selon la revendication 1, où ledit niveau préféré des mouvements pour le régime, qui est enregistré, est corrélé à l'indice de masse corporelle dudit être humain.

4. Agencement selon les revendications 1-3, où ladite rétroaction par l'intermédiaire de deux signaux au moins prescrit une augmentation ou une réduction des mouvements, respectivement.

5. Agencement selon la revendication 4, où lesdits signaux sont des sons, un affichage visuel ou des signaux de rétroaction tactiles.

6. Agencement selon les revendications 1-5, où ledit processeur et lesdits moyens sont logés dans un boîtier portable muni d'un affichage.

7. Agencement selon la revendication 6, où ledit boîtier comprend ledit un capteur au moins.

8. Agencement selon les revendications 1-7, où lesdits mouvements prédéterminés enregistrés diffèrent entre différentes activités.

9. Procédé utilisant un arrangement portable (10, 12) sur le corps, permettant de corriger le montant de l'activité physique à un niveau de régime alimentaire préféré, comprenant:
la fixation d'un capteur (12) au moins à une partie du corps d'un utilisateur humain, qui enregistre des mouvements avec une résolution prédéterminée du mouvement de ladite partie du corps;
le contrôle et l'enregistrement de signaux d'entrée fournis par ledit capteur (12) par un processeur auquel est reliée une mémoire;
la comparaison desdits signaux d'entrée à des mouvements prédéterminés à l'intérieur d'une résolution donnée, enregistrés dans ladite mémoire pour ledit niveau de régime; et
la mise à disposition d'une rétroaction par l'intermédiaire d'un signal de sortie fourni audit utilisateur, ledit signal de sortie indiquant comment lesdits mouvements sont à adapter auxdits mouvements enregistrés, adaptant ainsi l'activité physique du corps à un niveau qui correspond audit niveau de régime, de sorte que l'activité physique est corrélée audit niveau de régime.

10. Procédé selon la revendication 9, où lesdits mouvements enregistrés pour le niveau de régime préféré sont corrélés à l'un au moins parmi les paramètres poids et taille dudit être humain.

11. Procédé selon la revendication 9, où ledit niveau préféré des mouvements pour le régime,qui est enregistré, est corrélé à l'indice de masse corporelle dudit être humain.

12. Procédé selon les revendications 9-11, où ladite rétroaction par l'intermédiaire de deux signaux au moins prescrit une augmentation ou une réduction des mouvements, respectivement.

13. Procédé selon la revendication 12, où lesdits signaux sont des sons, un affichage visuel ou des signaux de rétroaction tactiles.

14. Procédé selon les revendications 9-13, où ledit processeur et lesdits moyens sont logés dans un boîtier portable muni d'un affichage.

15. Procédé selon la revendication 14, où ledit boîtier comprend ledit un capteur au moins.

16. Procédé selon les revendications 9-15, où lesdits mouvements prédéterminés enregistrés diffèrent entre différentes activités.
